# EUROPEAN PATENT SPECIFICATION

(11) **EP 1 817 079 B1**
(45) Date of publication and mention of the grant of the patent: **30.07.2008**
(21) Application number: 05813139.2
(22) Date of filing: 30.11.2005
(51) Int. Cl.: A61P 29/00

(54) **TREATMENT OF INFLAMMATION**
BEHANDLUNG VON ENTZÜNDUNGEN
TRAITEMENT ANTI-INFLAMMATOIRE

(30) Priority: 02.12.2004 US 632198 P; 03.03.2005 US 657718 P
(43) Date of publication of application: 15.08.2007
(73) Proprietor: Can-Fite Biopharma Ltd., Petach Tikva 49170 (IL)
(72) Inventor: FISHMAN, Pnina, 46365 Herzliya (IL); BAR-YEHUDA, Sara, 75474 Rishon Le Zion (IL)
(74) Representative: Hitchcock, Esmond Antony
(86) International application number: PCT/IL2005/001280
(87) International publication number: WO 2006/059328

(56) References cited:
- US-A1- 2004 167 094
- BAHARAV EHUD ET AL: "The anti-inflammatory effect of A3 adenosine receptor agonists in murine autoimmune arthritis models." INTERNATIONAL JOURNAL OF MOLECULAR MEDICINE, vol. 12, no. Supplement 1, 2003, page S42, XP009026428 & 8TH WORLD CONGRESS ON ADVANCES IN ONCOLOGY AND 6TH INTERNATIONAL SYMPOSIUM ON MOLECULAR MEDICINE; CRETE, GREECE; OCTOBER 16-18, 2003 ISSN: 1107-3756
- MABLEY JON G ET AL: "The A3 adenosine agonist, IB-MECA, protects against the development of arthritis and reverses established arthritis" FASEB JOURNAL, vol. 16, no. 5, 22 March 2002 (2002-03-22), page A1044, XP008060305 & ANNUAL MEETING OF PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- KRAUSE D: "Long-term combination therapy of rheumatoid arthritis: Methotrexate and parenteral gold" ZEITSCHRIFT FUER RHEUMATOLOGIE, vol. 57, no. 1, February 1998 (1998-02), pages 37-40, XP002369777 ISSN: 0340-1855
- BAHARAV EE ET AL: "Antiinflammatory effect of A3 adenosine receptor agonists in murine autoimmune arthritis models" JOURNAL OF RHEUMATOLOGY, TORONTO, CA, vol. 32, no. 3, 2005, pages 469-476, XP008060306 ISSN: 0315-162X
- ALETAHA D ET AL: "The rheumatoid arthritis patient in the clinic: comparing more than 1,300 consecutive DMARD courses." RHEUMATOLOGY (OXFORD, ENGLAND) DEC 2002, vol. 41, no. 12, December 2002 (2002-12), pages 1367-1374, XP002369778 ISSN: 1462-0324

## Description

### FIELD OF THE INVENTION

This invention relates to the field of therapeutics and in particular to the treatment of inflammatory conditions.

### PRIOR ART

The following is a list of art which is considered to be pertinent for describing the state of the art in the field of the invention. Acknowledgement of these references herein will at times be made by indicating their number within brackets from the list below.
1. Fishman P, et al. Evidence for involvement of Wnt signaling pathway in IB-MECA mediated suppression of melanoma cells. Oncogene, 21:4060-4064 (2002).
2. Fishman P, et al. Targeting the A3 adenosine receptor for cancer therapy: inhibition of Prostate carcinoma cell growth by A3AR agonist. Anticancer Res, 23:2077-2083 (2003).
3. Madi L, et al. A3 adenosine receptor activation in melanoma cells: association between receptor fate and tumor growth inhibition. J. Bio. Chem., 278:42121-42130 (2003).
4. Ohana G, et al. Inhibition of primary colon carcinoma growth and liver metastasis by the A3 adenosine receptor agonist IB-MECA. British J. Cancer., 89:1552-1558 (2003).
5. Fishman P, et al. An agonist to the A3 adenosine receptor inhibits colon carcinoma growth in mice via modulation of GSK-3β and NF-κB. Oncogene, 23:2465-2471 (2004).
6. US Patent Application publication No. 2004016709A1.
7. Szabo, C., et al. Suppression of macrophage inflammatory protein (MIP)-1α production and collagen-induced arthritis by adenosine receptor agonists. British J. Pharmacology, 125:379-387 (1998).
8. Mabley, J., et al. The adenosine A3 receptor agonist, N6-(3-iodobenzyl)-adenosine -5'-N-methyluronamide, is protective in two murine models of colitis. Europ. J. Pharmacology, 466:323-329 (2003).
9. Baharav, E., et al. The effect of adenosine and the A3 adenosine receptor agonist IB-MECA on joint inflammation and autoimmune diseases models. Intel. J. Mol. Med. 10(supplement 1) page S104, abstract 499 (2002).
10. Bahave E. et al. Antiinflammatory effect of A3 Adenosine receptor agonists in murine autoimmune arthritis models. J. Rehumatolog 32:469-476 (2005);
11. Montesinos, M. Carmen, et al. Adenosine A2A or A3 receptors are required for inhibition of inflammation by methotrexate and its analog MX-68. Arthritis & Rheumatism, 48:240-247 (2003).
12. Madi L, et al. The A3 Adenosine Receptor is Highly Expressed in Tumor vs. Normal Cells: Potential Target for Tumor Growth Inhibition. Clinical Cancer Research, 10: 4472-4479, (2004).
13. Gessi, S. et al. Elevated expression of A3 adenosine receptors in human colorectal cancer is reflected in peripheral blood cells Clinical Cancer Research 10:5895-5901, (2004).
14. US Patent Application No. 20040137477 A1.
15. Chan ES and Cronstein BN."Molecular action of methotrexate in inflammatory diseases ".Arthritis research. 4:266, (2002).
16. Montesinos MC; et al. Adenosine A2A or A3 receptors are required for inhibition of inflammation by methotrexate and its analog MX-68".Arthritis & rheumatism.48:240 (2003).
17. Yednock, TA. et al. Methods and compositions for treating rheumatoid arthritis, US Patent Application Publication No.20050074451.
18. Yednock, TA. et al. Methods and compositions for treating rheumatoid arthritis. US Patent Application Publication No.20050065192.
19. Feldman M. et al. Anti-TNF Antibodies and Methotrexate in the Treatment of Autoimmune Disease. AU200051825.
20. Alison MB et al. Combination therapy using a TNF binding protein for treating TNF-mediated diseases. US Patent Application Publication No. US2002119924.

### BACKGROUND OF THE INVENTION

### A₃ adenosine receptor

The A₃ adenosine receptor, a Gᵢ protein-associated cell surface receptor, has been proposed as a target to combat cancer and inflammation. The receptor is highly expressed in various tumor cell types while low expression was shown in adjacent normal tissues. Activation of the receptor by a specific synthetic agonist induces de-regulation of signal transduction pathways which include the Wnt and the NF-kB, resulting in tumor growth inhibition (1-5). *In vivo* studies have shown that A₃AR agonists inhibit the development of colon, prostate and pancreatic carcinomas as well as melanoma and hepatoma.

A₃AR agonists were also been shown to act as anti-inflammatory agents by ameliorating the inflammatory process in different experimental autoimmune models such as rheumatoid arthritis, Multiple sclerosis and Crohn's disease (6-10). It was proposed earlier that the A_{2A} and A₃ receptors mediate the anti-inflammatory effects of methotrexate (11).

A₃ adenosine receptor (A₃AR) expression levels are elevated in cancer cells as compared to normal cells (12, 13). Thus, the A₃AR expression level has been described as a mean for the diagnosis of cancer (14). In addition, A₃AR expression level has also been described to be elevated in peripheral blood mono-nuclear cells of patients with colorectal cancer (13).

### Methotrexate

Methotrexate (MTX) is a metabolic antagonist which was initially developed as therapeutic drug for malignant tumors and is currently used, in lower doses, for treating rheumatoid arthritis as well as other autoimmune and allergic diseases.

Although MTX was first introduced as an antiproliferative agent that inhibits the synthesis of purines and pyrimidines for the therapy of malignancies, it is now clear that many of the anti-inflammatory effects of methotrexate are mediated by adenosine (15). In confirmation of this mechanism of action, studies in both animals and patients suggested that adenosine-receptor antagonists, among which is caffeine, reverse or prevent the anti-inflammatory effects of methotrexate (15, 16).

Combination therapies comprising MTX and an additional agent have been suggested: for example, the combination of MTX with an antibody or an inhibitor of α-4-integrin (17, 18), and the combination of MTX with anti-TNF antibodies or TNF-binding protein in the treatment of TNF-mediated disorders, including autoimmune diseases (19, 20).

### SUMMARY OF THE INVENTION

The present invention is based on the finding that a combined treatment of the A3 adenosine receptor (A₃AR) agonist IB-MECA and MTX led to a greater anti-inflammatory effect in an inflammatory arthritis animal model than treatment with MTX alone or with an A₃AR agonist alone.

Thus, the present invention provided uses of an A₃ adenosine receptor or of methotrexate as claimed.

The A₃AR agonists, in accordance with the invention, are preferably, but not exclusively, N⁶- (3-iodobenzyl)-adenosine- 5'-N-methyl-uronamide (IB-MECA) and 2-chloro-N⁶- (3-iodobenzyl)-adenosine- 5'-N-methyl-uronamide (Cl-IB-MECA). However, these currently preferred A₃AR agonists are by no means exclusive and other such agonists may also be used, as detailed further below.

A preferred, but not exclusive, inflammatory condition to be treated in accordance with the invention is an autoimmune disorder, more preferably Rheumatoid Arthritis (RA).

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Figs. 1A-1B** are graphical representations of results of an experiment showing the change in severity of arthritis as a function of time in AIA animals treated with either methotrexate (MTX; 0.25 mg/Kg in **Fig. 1A** and 0.25 or 0.75 mg/Kg in **Fig. 1B****),** IB-MECA (CF101), or a combination of MTX and IB-MECA or with vehicle only (control).
**Figs. 2A-2B** are bar graphs showing the effect of combined treatment of IB-MECA (CF101) and MTX on the proliferation of human fibroblasts like synoviocytes (FLS) (**Fig. 2A**) or rat FLS (**Fig. 2B**) as determined by MTT assay.

### DETAILED DESCRIPTION OF THE INVENTION

The invention is described in the following detailed description with reference to therapeutic methods for the treatment of inflammatory conditions involving administration of a combination of an A₃AR agonists and MTX.

As used in the specification and claims, the forms "***a***", "***an***" and "***the***" include singular as well as plural references unless the context clearly dictates otherwise. For example, the term **"*an A₃AR agonist*"** includes one or more agonists.

Further, as used herein, the term **"*comprising*"** is intended to mean that the methods or composition includes the recited elements, but not excluding others. Similarly, "***consisting essentially of***" is used to define methods and compositions that include the recited elements but exclude other elements that may have an essential significance on the anti-inflammatory activity. For example, a composition consisting essentially of an A₃AR agonist will not include or include only insignificant amounts (amounts that will have an insignificant effect on the anti-inflammatory effect of the composition) of other active ingredients that have an anti-inflammatory activity. Also, a composition consisting essentially of the active agents as defined herein would not exclude trace contaminants from the isolation and purification method, pharmaceutically acceptable carriers, such as phosphate buffered saline, excipients, preservatives, and the like. "***Consisting of***" shall mean excluding more than trace elements of other elements. Embodiments defined by each of these transition terms are within the scope of this invention.

Further, all numerical values, e.g., concentration or dose or ranges thereof, are approximations which are varied (+) or (-) by up to 20%, at times by up to 10% of from the stated values. It is to be understood, even if not always explicitly stated that all numerical designations are preceded by the term "***about***". It also is to be understood, although not always explicitly stated, that the reagents described herein are merely exemplary and that equivalents of such are known in the art.

As detailed in the following exemplary embodiment, the invention is based on the finding that treatment of animals having induced inflammatory arthritis (Adjuvant induced Arthritis, AIA) with IB-MECA, an A₃AR agonist, in combination with MTX resulted in a combined anti-inflammatory effect, significantly larger than any of these drugs alone.

Thus, there is provided a method for the treatment of an inflammatory condition, the method comprises combined administering to a subject in need of said anti-inflammatory treatment of an effective amount of MTX and an effective amount of A₃AR agonist.

A combined administration denotes administering to a patient the A₃AR agonist and MTX within the same treatment course; namely during a treatment period, that can last weeks, months or years. Both active agents are given to the patients, each one according to its specific administration schedule. MTX is typically given to patients once weekly, either orally or parentally. An A₃AR agonist may be administered to a patient orally or parentally, once daily, twice daily, several times daily, every other day, etc. A combined treatment may involve, for example, weekly administration of MTX and once or twice daily administration of an A₃AR agonist. A combination treatment is a treatment involving the above combined administration.

A combined treatment may be indicated to patients not previously treated by either MTX or an A₃AR agonist, or to patients treated with either MTX or an A₃AR agonist that are either not responding properly to the existing treatment or for the purpose of amplifying the therapeutic response.

Thus, when referring to combined administrations, the invention also pertains to the use of an A₃AR agonist for the preparation of a pharmaceutical composition for treating an inflammatory disease which is to be treated with MTX, or alternatively, to the use of MTX for the preparation of a pharmaceutical composition for treating an inflammatory disease which is to be treated with an A₃AR agonist.

The combined treatment of the two active principles produces an anti-inflammatory effect. Herein, the term ***"anti-inflammatory*"** will be used to denote the disease modifying effect achieved by the combined treatment in alleviating the inflammatory response in inflammatory conditions. The anti-inflammatory response may be determined on the basis of various parameters as known to the practitioner. For example, when the inflammatory condition is arthritis, the parameters may include one or more of the following: histological parameters, blood parameters of inflammation, the extent of swollen and tender joints, motility parameters, reduction in pain, a number of different overall performance scoring systems, etc.

Thus, the term **"*inflammatory condition"*** in accordance with the invention shall mean any state of active or sub-clinical inflammatory condition, including immune induced pathologies, that is characterized by a persistent inflammatory response with pathologic sequelae. This state may be characterized by infiltration of mononuclear cells, proliferation of fibroblasts and small blood vessels, increased connective tissue, and tissue destruction.

The term **"*inflammatory condition*"** may include a variety of conditions associated with inflammatory responses and immune induced pathologies mediated (e.g. autoimmune disorders) by the immune system. However, the term **"*inflammatory condition*"** denotes such conditions in which treatment with MTX is currently indicated. Without being limited thereto, inflammatory conditions include psoriasis, psoriatic arthritis, Crohn's disease, rheumatoid arthritis as well as other rheumatic diseases, including polymyositis and systemic lupus erythematosus

As to the active principles, i.e. A₃AR agonists and MTX, both are known in the art. Generally, the A₃AR agonist is any compound that is capable of specifically binding to the adenosine A₃ receptor ("*A₃R*"), thereby fully or partially activating said receptor thereby yielding a therapeutic effect (in this particular case, an anti-inflammatory effect). The A₃AR agonist is thus a molecule that exerts its prime effect through the binding and activation of the A₃AR. This means that at the doses it is being administered it essentially binds to and activates only the A₃R.: As an example, the A₃AR agonist has a binding affinity (Kᵢ) to the human A₃AR of less than 1000 nM, desirably less than 500 nM, advantageously less 200 nM and even less than 100 nM, typically less than 50 nM, preferably less than 20 nM, more preferably less than 10 nM and ideally less than 5 nM. The lower the Kᵢ, the lower the dose of the A₃AR agonist (that may be used) that will be effective in activating the A₃R and thus achieving a therapeutic effect.

It should be noted that some A₃AR agonists can also interact with and activate other receptors with lower affinities (namely a higher Ki). A molecule will be considered an A₃AR agonists in the context of the publication (namely a molecule that exerts its prime effect through the binding and activation A₃R) if its affinity to the A₃R is at least 3 times (i. e. its Ki to the A₃R is at least 3 times lower), preferably 10 times, desirably 20 times and most preferably at least 50 times larger than the affinity to any other of the adenosine receptors.

The affinity of A₃AR agonists to the human A₃R as well as its relative affinity to the other human adenosine receptors can be determined by a number of assays, such as a binding assay. Examples of binding assays include providing membranes or cells having the receptor and measuring the ability of the A₃AR agonist to displace a bound radioactive agonist; utilizing cells that display the respective human adenosine receptor and measuring, in a functional assay, the ability of the A₃AR agonist to activate or deactivate, as the case may be, downstream signaling events such as the effect on adenylate cyclase measured through increase or decrease of the cAMP level; etc. Clearly, if the administered level of an A₃AR agonist is increased such that its blood level reaches a level approaching that of the Ki of the other adenosine receptors, activation of these receptors may occur following such administration, in addition to activation of the A₃R. An A₃AR agonist is thus preferably administered at a dose such that the blood level that will be attained will give rise to essentially only A₃R activation.

The characteristic of some adenosine A₃AR agonists and methods of their preparation are described in detail in, *inter alia,* US 5,688,774; US 5,773,423; US 5,573,772; US 5,443,836; US 6,048,865; WO 95/02604; WO 99/20284; WO 99/06053; and WO 97/27173.

According to one embodiment of the invention, the A₃AR agonist is a purine derivative falling within the scope of the general formula (I): 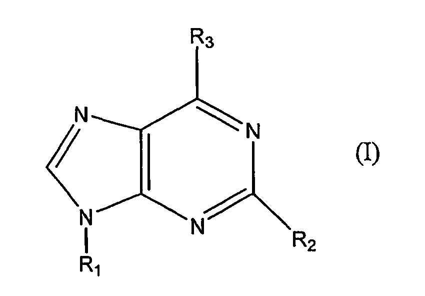 wherein R₁ is C₁-C₁₀ alkyl, C₁-C₁₀ hydroxyalkyl, C₁-C₁₀ carboxyalkyl or C₁-C₁₀ cyanoalkyl or a group of the following general formula (II): 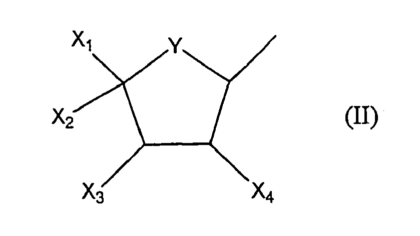 in which:
- Y is oxygen, sulfur atom or CH₂;
- X₁ is hydrogen, C₁-C₁₀ alkyl, R^{a}R^{b}NC(=O)- or HOR^{c}-, wherein R^{a} and R^{b} may be the same or different and are selected from hydrogen, C₁-C₁₀ alkyl, amino, C₁-C₁₀ haloalkyl, C₁-C₁₀ aminoalkyl, C₁-C₁₀ BOC-aminoalkyl, and C₃-C₁₀ cycloalkyl or are joined together to form a heterocyclic ring containing two to five carbon atoms, and R^{c} is selected from C₁-C₁₀ alkyl, amino, C₁-C₁₀ haloalkyl, C₁-C₁₀ aminoalkyl, C₁-C₁₀ BOC-aminoalkyl, and C₃-C₁₀ cycloalkyl;
- X₂ is hydrogen, hydroxyl, C₁-C₁₀ alkylamino, C₁-C₁₀ alkylamido or C₁-C₁₀ hydroxyalkyl;
- X₃ and X₄ each independently are hydrogen, hydroxyl, amino, amido, azido, halo, alkyl, alkoxy, carboxy, nitrilo, nitro, trifluoro, aryl, alkaryl, thio, thioester, thioether, -OCOPh, -OC(=S)OPh or both X₃ and X₄ are oxygen connected to >C=S to form a 5-membered ring, or X₂ and X₃ form the ring of formula (III): 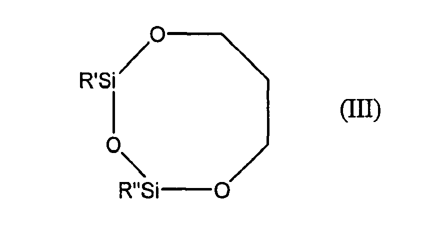
where R' and R" are independently C₁-C₁₀ alkyl;
- R₂ is selected from hydrogen, halo, C₁-C₁₀ alkylether, amino, hydrazido, C₁-C₁₀ alkylamino, C₁-C₁₀ alkoxy, C₁-C₁₀ thioalkoxy, pyridylthio, C₂-C₁₀ alkenyl; C₂-C₁₀ alkynyl, thio, and C₁-C₁₀ alkylthio; and
- R₃ is a NR₄R₅ group with R₄ being hydrogen or a group selected from alkyl, substituted alkyl or aryl-NH-C(Z)-, with Z being O, S, or NR^{a}, and
- when R₄ is hydrogen, R₅ being selected from R- and S-1-phenylethyl, benzyl, phenylethyl or anilide groups, each said groups being unsubstituted or substituted in one or more positions with a substituent selected from C₁-C₁₀ alkyl, amino, halo, C₁-C₁₀ haloalkyl, nitro, hydroxyl, acetoamido, C₁-C₁₀ alkoxy, and sulfonic acid or a salt thereof; or R₅ is benzodioxanemethyl, fururyl, L-propylalanyl- aminobenzyl, β-alanylamino- benzyl, T-BOC-β-alanylaminobenzyl, phenylamino, carbamoyl, phenoxy or C₁-C₁₀ cycloalkyl; or R₅ is a group of the following formula (IV): 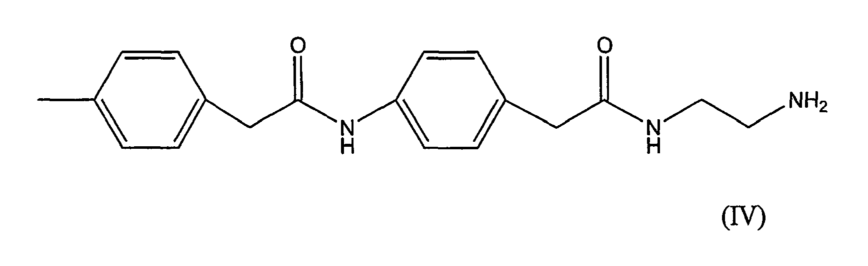 or, when R₄ is alkyl, substituted alkyl, or aryl-NH-C(Z)-, then, R₅ is selected from the group consisting of substituted or unsubstituted heteroaryl-NR^{a}-C(Z)-, heteroaryl-C(Z)-, alkaryl-NR^{a}-C(Z)-, alkaryl-C(Z)-, aryl-NR-C(Z)-and aryl-C(Z)-;
or the A₃AR agonist is a xanthine-7-riboside derivative of the following general formula (V): 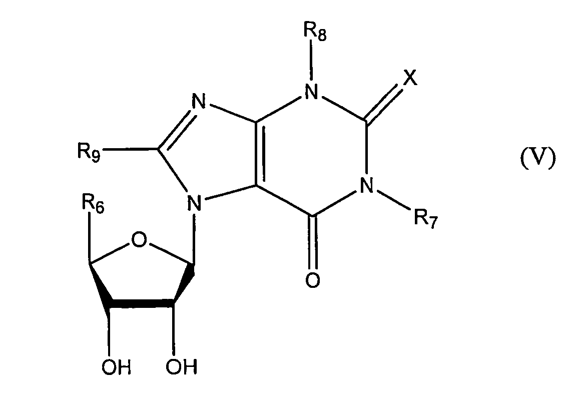 wherein:
- X is O or S;
- R₆ is R^{a}R^{b}NC(=O)- or HOP^{c}-, wherein
- R^{a} and R^{b} may be the same or different and are selected from hydrogen, C₁-C₁₀ alkyl, amino, C₁-C₁₀ haloalkyl, C₁-C₁₀ aminoakyl, and C₃-C₁₀ cycloalkyl, or are joined together to form a heterocyclic ring containing two to five carbon atoms; and
- R^{c} is selected from C₁-C₁₀ alkyl, amino, C₁-C₁₀ haloalkyl, C₁-C₁₀ aminoalkyl, C₁-C₁₀ BOC-aminoalkyl and C₃-C₁₀ cycloalkyl;
- R₇ and R₈ may be the same or different and are selected from C₁-C₁₀ alkyl, C₁-C₁₀ cycloalkyl, R- or S-1-phenylethyl, an unsubstituted benzyl or anilide group, and a phenylether of benzyl group substituted in one or more positions with a substituent selected from C₁-C₁₀ alkyl, amino, halo, C₁-C₁₀ haloalkyl, nitro, hydroxyl, acetamido, C₁-C₁₀ alkoxy, and sulfonic acid;
- R₉ is selected from the group consisting of halo, benzyl, phenyl, C₃-C₁₀ cyclalkyl, and C₁-C₁₀ alkoxy;
or a suitable salt of the compound defined above..

According to a more preferred embodiment, the A3AR agonist is a nucleoside derivative of the general formula (VII): 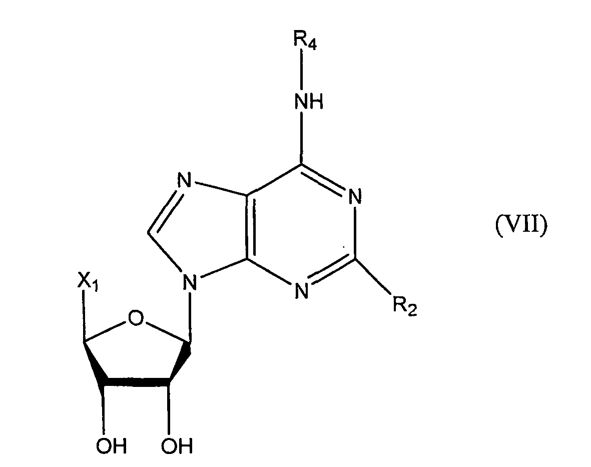 wherein X₁, R₂ and R₄ are as defined.

A specific group of A3AR agonists are the N⁶-benzyladenosine-5'-uronamide derivatives. Some preferred N⁶-benzyladenosine-5'-uronamide derivatives are N⁶-2-(4-aminophenyl)ethyladenosine (APNEA), N⁶-(4-amino-3-iodobenzyl) adenosine-5'-(N-methyluronamide) (AB-MECA) and 1-deoary-1-{6-[({3-iodophenyl} methyl)amino]- 9H-purine-9-yl}-N-methyl- β-D-ribofuranuronamide (IB-MECA) and 2-chloro-N⁶-(3-iodobenzyl)adenosine- 5'-N-methlyuronamide (C1-IB-MECA).

According to another embodiment, the A₃AR agonist is N⁶-benzyl-adenosine-5'-alkyluronamide-N¹-oxide or N⁶-benzyladenosine-5'-N-dialyl-uronamide-N¹oxide.

The MTX and A₃AR agonist are administered in amounts which are sufficient to achieve an anti-inflammatory effect. The amount of each active agent (MTX or A₃AR agonist) is at least the amount which provides the desired anti-inflammatory effect when given alone. Nonetheless, the use may be contemplated to provide therapeutic combinations that may lower total dosage of each active agent than may be required when each individual drug is used alone. A reduction in adverse effects may also be noted.

As will be appreciated, the amount of each of the active agents will depend on the condition to be treated, the intended therapeutic regiment and the desired therapeutic dose. By way of example, were the dose is 1 mg per day and the desired administration regiment is once daily administration, the amount of the active agent in a pharmaceutical composition comprising same will be 1 mg. where it is intended to divide this daily dose in 2 daily administrations, the amount of the active agent in the pharmaceutical composition will be 0.5 mg.

An amount effective to achieve the desired effect is determined by considerations known in the art. An **"*anti-inflammatory effective amount*"** for purposes herein must be effective to achieve any one of the following anti-inflammatory effect including, for example, amelioration of undesired symptoms associated with inflammation, prevention of the manifestation of such symptoms before they occur, slowing down progression of an inflammatory condition, slowing down the deterioration of symptoms associated with an inflammatory condition, slowing down any irreversible damage caused by a chronic stage of an inflammatory condition, lessening of the severity or curing an inflammatory condition, improving survival rate or providing more rapid recovery form such a condition.

By way of example, when the inflammatory condition is rheumatoid arthritis (RA, which is also a preferred condition to be treated by the present invention), the effective amount may be exhibited by one or more of the following effects: decreased swelling and tenderness of the joints, decreased in pain in the joints, improved motility and flexibility, slowing of the deterioration of the joints and the surrounding tissue, increase in the time period of the remission between acute attacks of the disease, decrease in the time period of the acute attack, prevention of the deterioration of the joints etc.

It is appreciated that the effective amount depends on a variety of factors including the affinity of the active agent to its corresponding receptor, its distribution profile within the body, a variety of pharmacological parameters such as half life in the body, on undesired side effects, if any, on factors such as age and gender of the subject to be treated, etc. The effective amount is typically tested in clinical studies having the aim of finding the effective dose range, the maximal tolerated dose and the optimal dose. The manner of conducting such clinical studies are well known to a person versed in the art of clinical development.

An amount may also at times be determined based on amounts shown to be effective in animals. It is well known that an amount of X mg/Kg administered to rats can be converted to an equivalent amount in another species (notably humans) by the use of one of possible conversions equations well known in the art. Examples of conversion equations are as follows:

### Conversion I:

| **Species** | **Body Wt. (Kg)** | **Body Surf. Area (m²)** | **Km Factor** |
|---|---|---|---|
| Mouse | 0.2 | 0.0066 | 3.0 |
| Rat | 0.15 | 0.025 | 5.9 |
| Human Child | 20.0 | 0.80 | 25 |
| Adult | 70.0 | 1.60 | 37 |

Body Surface area dependent Dose conversion: Rat (150g) to Man (70 Kg) is 1/7 the rat dose. This means that in the present case 0.001-0.4 mg/Kg in rats equals to about 0.14-56 microgram/Kg in humans; assuming an average weight of 70 Kg, this would translate into an absolute dosage of about 0.01 to about 4 mg.

### Conversion II:

The following conversion factors: Mouse = 3, Rat = 67. Multiply the conversion factor by the animal weight to go from mg/Kg to mg/m² for human dose equivalent.

| **Species** | **Weight (Kg)** | **BSA (m²)** |
|---|---|---|
| Human | 70.00 | 1.710 |
| Mouse | 0.02 | 0.007 |
| Rat | 0.15 | 0.025 |
| Dog | 8.00 | 0.448 |

According to this equation the amounts equivalent to 0.001-0.4 mg/Kg in rats for humans are 0.16-64 µg/Kg ; namely an absolute dose for a human weighing about 70 Kg of about 0.011 to about 4.4 mg, similar to the range indicated in Conversion I.

### Conversion III:

Another alternative for conversion is by setting the dose to yield the same plasma level or AUC as that achieved following administration to an animal.

Thus, based on measurement made in mice following oral administration of IB-MECA and based on such measurements made in humans in a clinical study in which IB-MECA was given to healthy male volunteers it was concluded that a dose of 1 microgram/Kg - 400 microgram/Kg in mice in which IB-MECA was effective and is equivalent to a human dose of about 0.14 - 57 microgram/Kg, namely a total dose for a 70 Kg individual of 0.01- 4 mg.

Further, based on the above conversion methods, a preferred dosage range for IB-MECA and Cl-IB-MECA (preferred A₃AR agonists in accordance with the invention) would be less than 4 mg, typically within the range of about 0.01 to about 2 mg (about 0.14 - 28 micrograms/Kg, respectively) and desirably within the range of about 0.1 to 1.5 mg (about 1.4 - 21 micrograms/Kg, respectively). This dose may be administered once, twice or at times several times a day.

Human studies as described in US patent application publication No. 20050101560 and by Fishman et al. [Fishman P. et al., Tolerability, pharmacokinetics, and concentration-dependent hemodynamic effects of oral CF101, an A3 adenosine receptor agonist, in healthy young men Int J Clin Pharmacol Ther. 42:534-542, 2004] showed that the level of IB-MECA decays in the human plasma with a half life of about 8-10 hours, as compared to a half life of only 1.5 hours in mice, in case of multiple daily administration, correction in the dosages for accumulative effects needs to be made at times (a subsequent dose is administered before the level of a previous one was decayed and thus, there is a build-up of plasma level over that which occurs in a single dose. On the basis of said human trials twice daily administration appears to be a preferred administration regiment. However this does not rule out other administration regiments.

Further, by way of example, an effective amount of MTX is typically in the range of 5 to 25 mg, administered once weekly, orally or parentally.

The combined treatment includes weekly administration to a subject of MTX, the dose being in the range between about 5 to 25 mg, concomitant with daily administration, between once and a few times a day, preferably once or twice a day, of A₃AR agonist, the dose in each administration being in the range of between about 1 to about 1000µg/kg body weight, preferably less than 400µg/kg body weight, and even less than 200µg/kg body weight. Typically, the dose of A₃AR agonist is in a range of 1 to 100 µg/kg body weight.

A **"*composition*"** is intended to mean a combination of the active agent(s), together or separately, with a pharmaceutically acceptable carrier as well as other additives. The carrier may at times have the effect of the improving the delivery or penetration of the active ingredient to the target tissue, for improving the stability of the drug, for slowing clearance rates, for imparting slow release properties, for reducing undesired side effects etc. The carrier may also be a substance that stabilizes the formulation (e.g. a preservative), for providing the formulation with an edible flavor, etc. For examples of carriers, stabilizers and adjuvants, see E.W. Martin, REMINGTON'S PHARMACEUTICAL SCIENCES, MacK Pub Co (June, 1990).

MTX and the A₃AR agonist may be administered to the subject by a variety of delivery modes as known in the art. While MTX may be administered orally or by parenteral injection, it is preferable that the A₃AR agonist be administered orally. The carrier will be selected based on the desired form of the formulation and the A₃AR agonist composition may be in the form of a pill, capsule, in the form of a syrup, an aromatic powder, and other various forms.

### DETAILED DESCRIPTION OF EXEMPLARY EMBODIMENTS

### EXAMPLE 1: In vivo Studies

### Materials

The A3AR agonist, CF101, a GMP grade of the compound known generically as 1-deoxy-1-[6-[[(3-iodophenyl)methyl]amino]-9H-purine-9-yl]-N-methyl-D-ribofuranuronamide (IB-MECA), was synthesized by Albany Molecular Research Inc. (Albany, NY, USA).

Methotrexate was purchased from Abic, Israel.

### Methods

Female Lewis rats, aged 8-12 weeks were obtained from Harlan Laboratories (Jerusalem, Israel). Rats were maintained on a standardized pelleted diet and supplied with tap water. Experiments were performed in accordance with the guidelines established by the Institutional Animal Care and Use Committee at Can-Fite BioPharma, Petah Tikva, Israel. The rats were injected subcutaneously (SC) at the tail base with 100 µl of suspension composed of incomplete Freund's adjuvant (IFA) with 10 mg/ml heat killed Mycobacterium tuberculosis, (Mt) H37Ra, (Difco, Detroit, USA). Each group contained 10 animals.

Treatment with CF101 (10 µg/kg) was initiated on day 14 after vaccination and was orally administered by gavage, twice daily. Another group was treated with Methotrexate (MTX) (0.25, 0.75 mg/kg) intraperitoneally every 3 days, starting on day 14th after vaccination. The control group in each experiment received vehicle only (DMSO in a dilution corresponding to that of the drugs).

Clinical Disease Activity Score was assessed as follows: the animals were inspected every second day for clinical arthritis. The scoring system ranged from 0-4 of each limb: 0- no arthritis; 1- redness or swelling of one toe/finger joint; 2-redness and swelling of more than one toe/finger joints, 3-the ankle and tarsal-metatarsal joints involvement. 4- entire paw redness or swelling. The clinical score was calculated by adding the four individual legs' score. The inflammatory intensity was also determined in accordance with the increase in the rat hind paw's diameter, measured by caliper (Mitotoyo, Tokyo, Japan).

### Results

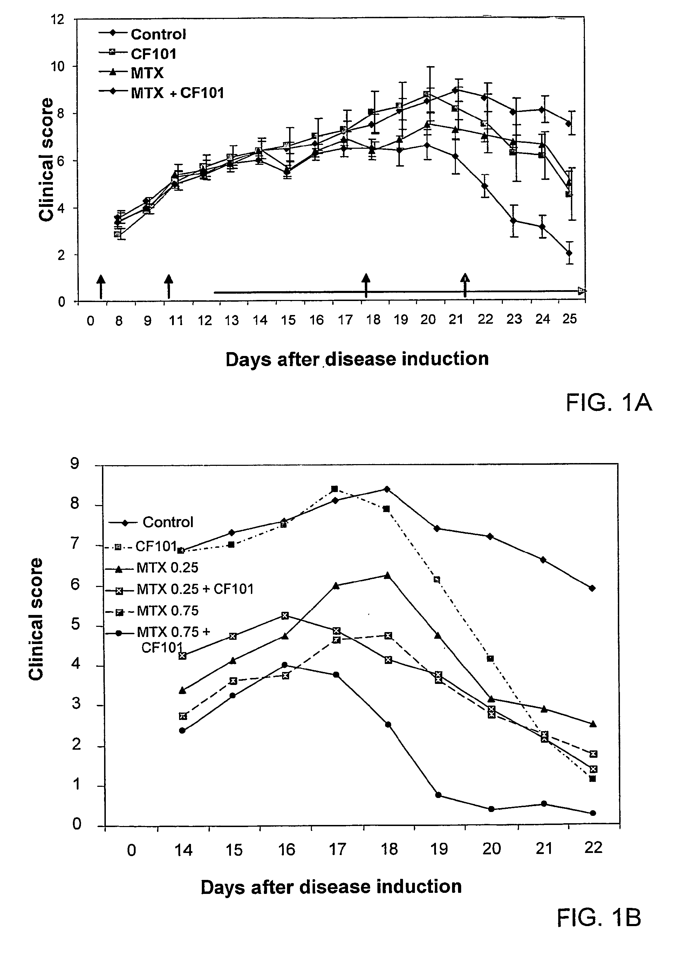

About 21 days after immunization, most of the vehicle treated animals progressively developed arthritis. CF101 treatment (10 µg/kg, given orally twice daily, starting on day 14th after immunization) and methotrexate (MTX) treatment resulted in a significant decrease in disease severity, very similar for both drugs, as was evaluated by the arthritis clinical score. Disease peaked on days 21-25 and maximal effect of CF 101 or MTX was seen on these days (**Fig. 1A and Fig. 1B**). A combined treatment with CF101 and MTX resulted in an inhibitory effect which is greater than the additive effect obtained with treatment of CF101 alone or MTX alone **(****Fig. 1A and Fig. 1B****).**

### EXAMPLE 2: In Vitro Studies

### Hunian Fibroblast Like Synoviocytes (FLS) cultures

Human synovial fluid samples were collected from osteoarthritis (OA) patients undergoing paracenthesis. The fluid was centrifuged and the supernatant removed. The cells were resuspended in DMEM containing type I collagenase (4mg/ml), for 2 hours, and shacked vigorously at 37°. The released cells in the supernatant were harvested by centrifugation and were cultured in DMEM containing 10% FBS, 2mM glutamine, 100 U/ml penicillin, 100µg/ml streptomycin, 1% non essential amino acids, 1% sodium pyruvate and 20 nM HEPES buffer in a 37°C, 5% CO₂ incubator. After overnight culture, non-adherent cells were removed. The adherent cells (FLS) were subcultured at a 1 : 2 ratio, and the cells from passages 4 through 10 were used in the experiments.

The effect of CF101 in combination with Methrotrexate (MTX) on the proliferation of the FLS was tested utilizing an MTT assay. The cells (5x10⁴/ml cells) were incubated in the presence of MTX (1 µM) in 96-well microtiter plates for 72 hours in the growth medium. At the last 24 hours CF101 (10nM) were added to the cultures.

### Rat Fibroblast Like Synoviocytes (FLS) cultures

Synovia tissue from adjuvant induced arthritis rats was collected. The tissue was minced and subjected for digestion in 4mg/ml type I collagenase and 0.25 w/v trypsine in DMEM. The mixture was shacked vigorously for 4 hours at 37°. The released cells were separated from the supernatant by centrifugation and cultured in DMEM containing 15% FCS, 2mM glutamine, 100 U/ml penicillin, 100µg/ml streptomycin in a 37°C, 5% CO₂ incubator. After overnight incubation the nonadherent cells were removed. The adherent cells (FLS) were sub-cultured at a 1 : 2 ratio, and the cells from passages 4 through 10 were used in the experiments.

The effect of CF101 in combination with MTX on the proliferation of the FLS was tested utilizing an MTT assay. The cells (5x10⁴/ml cells) were incubated in the presence of MTX (1 µM) in 96-well microtiter plates for 72 hours in the growth medium. At the last 24 hours CF 101 (10nM) were added to the cultures.

### Results

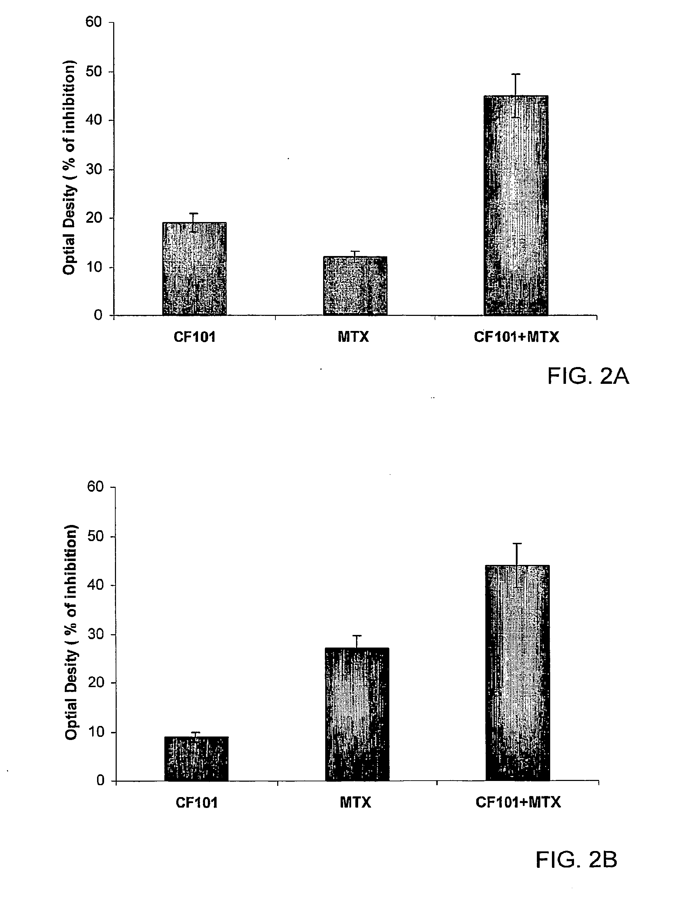

**Figs. 2A** and **2B** show, respectively, the effect of MTX alone, CF101 alone or combined treatment with MTX and CF101 on the proliferation of human and rat FLS, as evaluated by the MTT assay. As shown, the inhibitory effect of the combined treatment was greater than the effect achieved by each treatment alone.

## Claims

1. Use of an A₃ adenosine receptor (A₃AR) agonist for the preparation of a pharmaceutical composition for treating an inflammatory condition which is to be treated with methotrexate (MTX).

2. Use according to Claim 1, for the preparation of a pharmaceutical composition for treating an inflammatory condition which is to be treated with once weeldy MTX administration.

3. Use according to Claim 1 or 2, for the preparation of a pharmaceutical composition for 1 to several times daily administrations.

4. Use of MTX for the preparation of a pharmaceutical composition for treating an inflammatory condition which is to be treated with an A₃AR agonist.

5. Use according to Claim 4, for the preparation of a pharmaceutical composition for once weekly administration.

6. Use according to Claim 4 or 5, for the preparation of a pharmaceutical composition for treating an inflammatory condition which is to be treated between 1 and several times daily with said agonist.

7. Use according to any one of Claims 1 to 6, wherein said inflammatory condition is an autoimmune disorder.

8. Use according to Claim 7, wherein said autoimmune disorder is rheumatoid arthritis.

9. Use according to any one of Claims 1. to 8, wherein said A₃AR agonist is IB-MBCA.

## Patentansprüche

1. Verwendung eines A₃-Adenosinrezeptor-(A₃AR-)Agonisten für die Herstellung einer pharmazeutischen Zusammensetzung zur Behandlung eines Entzündungszustands, der mit Methotrexat (MTX) zu behandeln ist.

2. Verwendung nach Anspruch 1 für die Herstellung einer pharmazeutischen Zusammensetzung zur Behandlung eines Entzündungszustands, der mit einer einmaligen wöchentlichen MTX-Verabreichung zu behandeln ist.

3. Verwendung nach Anspruch 1 oder 2, zur Herstellung einer pharmazeutischen Zusammensetzung zur ein- bis mehrmaligen täglichen Verabreichung.

4. Verwendung von MTX für die Herstellung einer pharmazeutischen Zusammensetzung zur Behandlung eines Entzündungszustands, der mit einem A₃AR-Agonisten zu behandeln ist.

5. Verwendung nach Anspruch 4, für die Herstellung einer pharmazeutischen Zusammensetzung zur einmaligen wöchentlichen Verabreichung.

6. Verwendung nach Anspruch 4 oder 5 für die Herstellung einer pharmazeutischen Zusammensetzung zur Behandlung eines Entzündungszustands, der ein- bis mehrmals täglich mit dem Agonisten zu behandeln ist.

7. Verwendung nach einem der Ansprüche 1 bis 6, wobei der Entzündungszustands eine Autoimmunerkrankung ist.

8. Verwendung nach Anspruch 7, wobei die Autoimmunerkrankung Rheumatoidarthritis ist.

9. Verwendung nach einem der Ansprüche 1 bis 8, wobei der A₃AR-Agonist IB-MECA ist.

## Revendications

1. Utilisation d'un agoniste des récepteurs de l'adénosine A₃ (A₃AR) pour la préparation d'une composition pharmaceutique pour traiter une affection inflammatoire qui doit être traitée avec du méthotrexate (MTX).

2. Utilisation selon la revendication 1, pour la préparation d'une composition pharmaceutique pour traiter une affection inflammatoire qui doit être traitée avec une administration de MTX une fois par semaine.

3. Utilisation selon la revendication 1 ou 2, pour la préparation d'une composition pharmaceutique pour une à plusieurs administrations par jour.

4. Utilisation de MTX pour la préparation d'une composition pharmaceutique pour traiter une affection inflammatoire qui doit être traitée avec un agoniste de A₃AR.

5. Utilisation selon la revendication 4, pour la préparation d'une composition pharmaceutique à administrer une fois par semaine.

6. Utilisation selon la revendication 4 ou 5, pour la préparation d'une composition pharmaceutique pour traiter une affection inflammatoire qui doit être traitée entre 1 et plusieurs fois par jour avec ledit agoniste.

7. Utilisation selon l'une quelconque des revendications 1 à 6, dans laquelle ladite affection inflammatoire est un trouble auto-immun.

8. Utilisation selon la revendication 7, dans laquelle ledit trouble auto-immun est une polyarthrite rhumatoïde.

9. Utilisation selon l'une quelconque des revendications 1 à 8, dans laquelle ledit agoniste de A₃AR est IB-MECA.
